Europäisches Patentamt

European Patent Office

Office européen des brevets

(19)

(11) Veröffentlichungsnummer: **0 115 480 B1**

(12) **EUROPÄISCHE PATENTSCHRIFT**

(45) Veröffentlichungstag der Patentschrift:
29.10.86

(21) Anmeldenummer: **84890012.2**

(22) Anmeldetag: **16.01.84**

(51) Int. Cl.⁴: **A 61 B 5/14**, B 01 L 3/14

(54) **Blutprobenröhrchen.**

(30) Priorität: **27.01.83 AT 272/83**

(43) Veröffentlichungstag der Anmeldung:
**08.08.84 Patentblatt 84/32**

(45) Bekanntmachung des Hinweises auf die Patenterteilung:
**29.10.86 Patentblatt 86/44**

(84) Benannte Vertragsstaaten:
**BE CH DE FR GB IT LI LU NL SE**

(56) Entgegenhaltungen:
**EP - A - 0 059 297**
**DE - A - 2 243 593**
**US - A - 3 615 222**
**US - A - 3 682 315**
**US - A - 4 204 606**
**US - A - 4 303 067**
**US - A - 4 338 764**

(73) Patentinhaber: **C.A. GREINER & SÖHNE GESELLSCHAFT M.B.H., Greinerstrasse 70, A-4550 Kremsmünster Oberösterreich (AT)**

(72) Erfinder: **Konrad, Franz, in Preising 106, A-4844 Regau Oberösterreich (AT)**

(74) Vertreter: **Krause, Ernst, Dipl.-Ing. et al, Dipl.- Ing. Krause, Ernst Dipl. Ing. Casati, Wilhelm Patentanwälte Amerlingstrasse 8, A-1061 Wien (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein mit einer Dichtung verschlossenes, evakuierbares Blutprobenröhrchen für ein Blutabnahmegerät mit einem hohlzylindrischen Halter, der an einem Ende eine doppelendige Hohlnadel aufweist und in dessen anderes, offenes Ende das Blutprobenröhrchen soweit einschiebbar ist, dass das eine Nadelende die Dichtung durchstösst, wobei das Röhrchen an seinem offenen Ende durch ein Verschlusselement, z.B. aus einer Aluminiumfolie, abgedeckt ist, das zusätzlich aussen eine Dichtungsmembran aus gummielastischem Werkstoff, z.B. Silikonkautschuk, trägt und aus einer ebenen, vorzugsweise gleichbleibende Wandstärke aufweisenden Folie besteht, die an ihrer dem Röhrcheninneren zugewandten Seite mit einer durchgehenden heissiegelbaren Schicht versehen ist.

Ein Röhrchen für ein Vakuum-Blutentnahmesystem ist aus der DE-OS 29 08 817 bekannt. Das Verschlusselement bildet dort eine aufschraubbare Kappe, die eine Öffnung aufweist, deren Rand eine die Dichtung bildende Membrane aus einem Material, das vom einen Ende der Nadel durchstechbar ist, an das offene Ende des Blutprobenröhrchens abdichtend andrückt. Bei diesem Röhrchen müssen also an seiner Öffnung und an seiner Verschlusskappe Gewinde hergestellt werden, und ferner muss beim Aufschrauben der Kappe eine sorgfältige Dichtung der Membrane zwischen der Kappe und dem Röhrchenende hergestellt werden. Der Zugang zu der mit einem solchen Röhrchen entnommenen Probe erfordert letztlich wieder das Abschrauben der Kappe, was umständlich und zeitraubend ist; ausserdem kann am Röhrchen nicht festgestellt werden, ob ein ungewolltes Öffnen vorgenommen ist. Ausserdem hat sich herausgestellt, dass bei längerer Lagerung dieser Röhrchen, insbesondere wenn sie aus mikroporösem Kunststoff bestehen, das Vakuum nicht mehr oder nur mehr ungenügend vorhanden ist.

Ein Röhrchen ähnlich der eingangs genannten Art ist aus der DE-OS 22 43 593 bekannt. Bei einer besonderen Ausführungsform wird dabei eine nach Art eines Kronenverschlusses geformte Metallkappe vorgesehen, die in ihrem Mittelteil einen kreisförmigen Kronenteil und daran nach aussen anschliessend eine ringförmige herabhängende Manschette mit umgekehrtem U-Profil aufweist, das in einen nach oben umgekröpften Rand endet. Der Mittelteil des kreisförmigen Kronenteiles ist mit geringerer Dicke ausgebildet, um leicht von der Hohlnadel durchstochen werden zu können. Wenigstens die Innenfläche der Metallkappe, vorzugsweise ihre gesamte Unterfläche, ist dabei mit einem dünnen Schutzüberzug aus Vinyl überzogen, um eine Verunreinigung des Röhrcheninhaltes hintanzuhalten. Die Dichtung ist an der Aussenfläche der Metallkappe befestigt, und die Festlegung der Kappe mit ihrem U-Profil am Röhrchenende erfolgt mit Hilfe eines heissschmelzenden Materials, wie Polyamid, das eine Klebebindung zwischen der Stossfläche der Manschette und der Aussenfläche des Rohrendes bildet. In dieser Weise kann die Metallkappe mit einem Kronenkorkenöffner abgenommen werden. Auch diese bekannte Lösung weist nicht nur eine Vielzahl von kompliziert geformten bzw. herzustellenden Einzelteilen auf, sondern auch einen umständlichen Aufwand beim Zusammenbau und bei der Handhabung.

Das Röhrchen der letzterwähnten Art wird nach der AT-PS 368 389 dadurch verbessert, dass der Verschluss aus einer ebenen, vorzugsweise gleichbleibende Wandstärke aufweisenden Folie besteht, die einseitig mit einer durchgehenden heissiegelbaren Schicht versehen ist, welche eine dichte Verbindung zwischen der Folie und der Endstirnfläche des Röhrchens bildet und den Röhrcheninnenraum gegen die Folie isoliert.

Von diesem bekannten Stand der Technik ausgehend, stellt sich die Erfindung die Aufgabe, die Übelstände bei den Röhrchen der beiden erstgenannten Arten zu beseitigen und dabei ein Verschlusselement zu schaffen, das leicht zu öffnen ist und beim Durchstechen der Membrane mit der doppelendigen Nadel einen möglichst geringen Widerstand leistet.

Die Lösung dieser Aufgabe besteht gemäss der Erfindung darin, dass das Verschlusselement mittels der heissiegelbaren Schicht an einem Flansch einer einen Doppelmantel aufweisenden Kappe festgelegt ist, die das offene Ende des Röhrchens von innen und aussen umfasst und mittels Presssitz in diesem gelagert ist. Nach dem Wesen der Erfindung ist sohin eine Kappe mit Doppelmantel vorgesehen, die an das Ende des Röhrchens aufgesteckt ist, und es wird durch den Doppelmantel eine besonders gute Abdichtung zum Röhrchen erzielt, sodass damit Vakuumverluste vermieden werden. Weiters ist von Vorteil, dass man mit dieser Kappe wiederum sehr leicht durch einfaches Eindrücken in das Röhrchen die Blutproben verschliessen kann und dass damit ein verschlossenes Aufbewahren ermöglicht wird.

Ein weiteres Merkmal der Erfindung ist, dass der Flansch etwa in der halben Höhe des inneren Kappenmantels angeordnet ist. In dieser Weise vermag der Flansch den gewünschten Presssitz der Kappe am Röhrchen zu unterstützen.

Ein anderes Merkmal der Erfindung sieht vor, dass der äussere Kappenmantel länger ist als der innere Kappenmantel. Damit wird verhindert, dass der Benützer beim Öffnen des Röhrchens mit dem mit Blut benetzten inneren Mantel nicht in Berührung kommen kann und damit eine Erkrankung durch infektiöses Blut verhindert wird.

Weitere Einzelheiten der Erfindung ergeben sich aus der folgenden Beschreibung eines Ausführungsbeispieles anhand der Zeichnung, in der

Fig. 1 eine den Verschluss tragende Kappe im Vertikalschnitt und

Fig. 2 diese Kappe in ähnlicher Weise, aufgesteckt auf ein Blutprobenröhrchen, je zur Hälfte in Seitenansicht, darstellen.

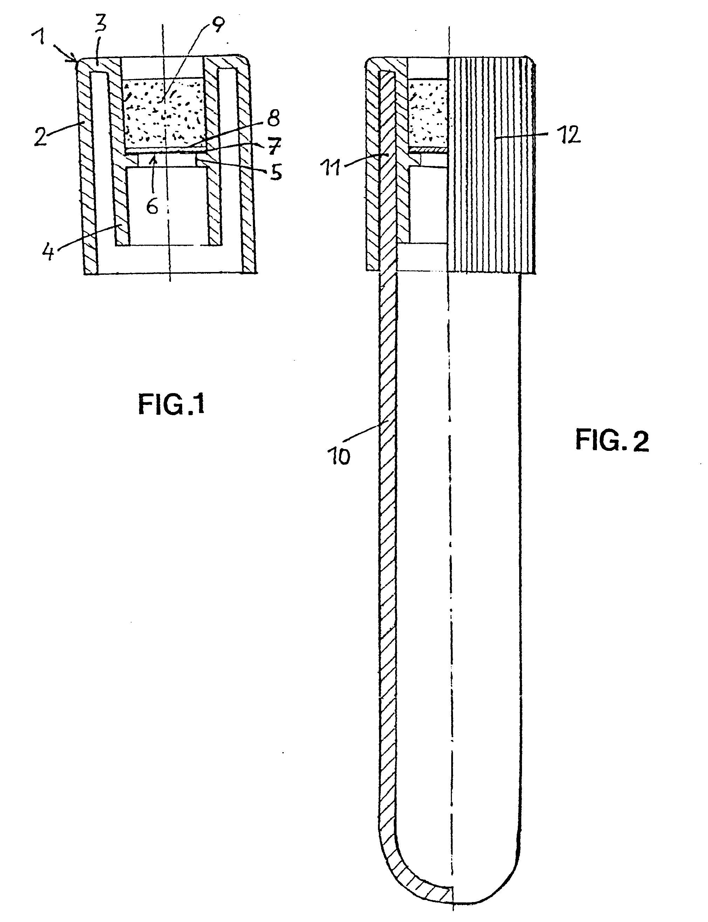

Die aus Kunststoff bestehende Kappe 1, hier zylindrischer Form, weist, wie aus Fig. 1 hervorgeht, einen äusseren Mantel 2, eine Stirnwand 3 und einen inneren Mantel 4 auf. Dieser innere Mantel 4 ist innen in halber Höhe mit einem Flansch 5 versehen, der an seiner Oberseite einen Verschluss 6 trägt, der in Form einer Platine aus einer mit einer Heissiegelschicht 7 versehenen, gleichbleibenden Wandstärke aufweisenden Aluminiumfolie 8 ausgebildet ist. Solche Folien sind an sich bekannt. Zweckmässig wird eine 0,3 mm starke Aluminiumfolie verwendet.

Auf der Folie 8 ist ferner ein etwa 1,5–3 mm starkes Dichtungspolster 9 vorgesehen, das aus einem gummielastischem Werkstoff, vorzugsweise Silikonkautschuk, besteht.

Dieses luftdicht aufvulkanisierte Dichtungspolster hat die Eigenschaft, dass es sich, wenn man das eine Ende der doppelendigen Nadel des hohlzylindrischen Halters hindurchsticht und die Nadel nach der Blutabnahme wieder herauszieht, wieder ganz schliesst.

Wie aus Fig. 2 erkenntlich, ist das zu verschliessende Röhrchen 10 mit seinem offenen Ende 11 zwischen dem äusseren Mantel 2 und dem inneren Mantel 4 eingeschoben, unter Herstellung eines Pressitzes zwischen diesen Teilen. Die Aussenseite des Aussenmantels 2 kann, wie dargestellt, mit Riefen 12 versehen sein.

Der Innenraum des Röhrchens 10 steht unter Vakuum; dieses kann nach dem Heissiegeln des Verschlusses 6 und dem Aufbringen des Dichtungspolsters 9 erzeugt werden.

Die Handhabung und Wirkungsweise dieses Röhrchens bezüglich der Blutabnahme ist wie in der genannten DE-OS 2 908 817 beschrieben.

An dem beschriebenen Ausführungsbeispiel können Abänderungen vorgenommen werden. So kann das Röhrchen 10 z.B. auch aus Kunststoff oder Glas bestehen. Es ist ferner möglich, den Verschluss 6 auch aus einer Kunststofffolie 8 herzustellen.

Das erfindungsgemäss ausgebildete Röhrchen kann nicht nur zur Blutentnahme, sondern auch zur Entnahme von anderen Körperflüssigkeiten und auch für dazu ähnliche Zwecke eingesetzt werden.

## Patentansprüche

1. Mit einer Dichtung verschlossenes, evakuierbares Blutprobenröhrchen für ein Blutabnahmegerät mit einem hohlzylindrischen Halter, der an einem Ende eine doppelendige Hohlnadel aufweist und in dessen anderes, offenes Ende das Blutprobenröhrchen soweit einschiebbar ist, dass das eine Nadelende die Dichtung durchstösst, wobei das Röhrchen an seinem offenen Ende durch ein Verschlusselement, z.B. aus einer Aluminiumfolie, abgedeckt ist, das zusätzlich aussen eine Dichtungsmembran aus gummielastischem Werkstoff, z.B. Silikonkautschuk, trägt und aus einer ebenen, vorzugsweise gleichbleibende Wandstärke aufweisenden Folie besteht, die an ihrer dem Röhrcheninneren zugewandten Seite mit einer durchgehenden heissiegelbaren Schicht versehen ist, dadurch gekennzeichnet, dass das Verschlusselement (6) mittels der heissiegelbaren Schicht (7) an einem Flansch (5) einer einen Doppelmantel (2, 4) aufweisenden Kappe (1) festgelegt ist, die das offene Ende (11) des Röhrchens (10) von innen und aussen umfasst und mittels Pressitz in diesem gelagert ist.

2. Röhrchen nach Anspruch 1, dadurch gekennzeichnet, dass der Flansch (5) etwa in der halben Höhe des inneren Kappenmantels (4) angeordnet ist.

3. Röhrchen nach Anspruch 1 oder 2, dadurch gekennzeichnet, dass der äussere Kappenmantel (2) länger ist als der innere Kappenmantel (4).

## Revendications

1. Tube de prélèvement du sang susceptible d'être placé sous vide et obturé par une fermeture étanche, destiné à un appareil de prise de sang avec un support cylindrique creux, qui comporte, à une extrémité, une aiguille creuse à deux bouts et dans l'autre extrémité ouverte duquel est intercalable le tube de prélèvement du sang de telle sorte que l'une des extrémités de l'aiguille perce la fermeture étanche, le tube étant recouvert, à son extrémité ouverte, d'un élément de fermeture (par exemple une feuille d'aluminium) qui supporte en outre à l'extérieur une membrane d'étanchéification en matière caoutchouteuse élastique, par exemple en caoutchouc de silicone, et est une feuille lisse, de préférence d'épaisseur constante, qui est équipée, sur sa face tournée vers l'extérieur du tube, d'une couche continue scellable à chaud, caractérisé en ce que l'élément de fermeture (6) est fixé au moyen de la couche scellable à chaud (7) à une collerette (5) d'un capuchon (1) présentant une double enveloppe (2,4) qui entoure, par l'intérieur et par l'extérieur, l'extrémité ouverte (11) du tube (10) et est montée dans ce dernier par un ajustage serré.

2. Tube de prélèvement du sang selon la revendication 1, caractérisé en ce que la collerette (5) est disposée environ à mi-hauteur de l'enveloppe intérieure (4) du capuchon.

3. Tube de prélèvement du sang selon la revendication 1 ou la revendication 2, caractérisé en ce que l'enveloppe extérieure (2) du capuchon est plus longue que l'enveloppe intérieure (4).

## Claims

1. Test tube for blood samples for a blood removing device, which tube is closed by a seal and adapted to be evacuated and includes a hollow cylindrical holder which has a double-ended hollow needle at one end and into whose other, open end the test tube may be slid in so far that one end of the needle pierces the seal, whereby the test tube is covered at its open end by a closure element, e.g. of aluminium foil, which additionally carries on its exterior a sealing membrane of rubberelastic material, e.g. silicon rubber, and comprises a flat foil, preferably of constant thickness, which is provided on its surface directed towards the interior of the test tube with a continuous heat-sealable layer, characterised in that the closure element (6) is fixed by means of the heat-

5 0 115 480 6

sealable layer (7) to a flange (5) of a cap (1) having a double wall (2, 4), which cap surrounds the open end (11) of the test tube (10) from the inside and the outside and is press fitted in it.

2. Test tube as claimed in claim 1, characterised in that the flange (5) is disposed about half way up the inner wall (4) of the cap.

3. Test tube as claimed in claim 1 or 2, characterised in that the outer wall (2) of the cap is longer than the inner wall (4) of the cap.

4

FIG.1

FIG. 2